# EUROPEAN PATENT APPLICATION

(11) **EP 1 500 934 A1**
(43) Date of publication of application: **26.01.2005**
(21) Application number: 02722738.8
(22) Date of filing: 24.04.2002
(51) Int. Cl.: G01N 33/577, G01N 33/569

(54) **A METHOD FOR DETERMINING A RISK OF ATHEROSCLEROSIS OR FUTURE DEVELOPMENT OF ATHEROSCLEROTIC COMPLICATIONS**

(71) Applicant: WAKO PURE CHEMICAL INDUSTRIES, LTD, Chuo-ku Osaka 541 (JP)
(72) Inventor: TAKAHASHI, Hakuo, Otsu-shi, Shiga 520-0016 (JP); MASUDA, Midori, Suma-ku, Kobe-shi, Hyogo 654-0009 (JP)
(74) Representative: Winter, Brandl, Fürniss, Hübner, Röss, Kaiser, Polte Partnerschaft
(86) International application number: PCT/JP2002/004067
(87) International publication number: WO 2003/091733

(57) **Abstract**

The present invention discloses a method for determining a risk of atherosclerosis or future development of atherosclerotic complications by measuring an amount of a soluble Fcγ receptor IIIa^{Mφ} derived from macrophage (hereafter abbreviated as sFcγRIIIa^{Mφ}) in a blood sample from patients by using an antibody specifically recognizing FcγRIIIa^{Mφ}, and determining the risk on the basis of the result of the measurement, a kit for measuring an amount of sFcγRIIIa^{Mφ} and a kit for determining a risk of development of atherosclerosis or future development of atherosclerotic complications.

A determination method of the present invention is a non-invasive method with greatly mitigated patient burden compared with a conventional cardiac catheterization method, because it is a method by measuring an amount of sFcγRIIIa^{Mφ} in a blood sample, which is a novel predictive marker of atherosclerosis. Furthermore, the present method can also determine a risk of coming down with atherosclerotic complications in the future for a case of superficially healthy but with progressing atherosclerosis internally.

## Description

### TECHNICAL FIELD

The present invention relates to a method for determining a risk of atherosclerosis or future development of atherosclerotic complications on the basis of measuring the circulating level of soluble Fcγ receptor IIIa^{Mφ}.

### BACKGROUND ART

Recently, atherosclerotic complications have been increasing progressively with the aged society, and it has been providing a serious social problem not only due to the increased medical costs but also due to loss of work force. That is, people afflict most of the period between healthy life and death with a handicap by complications such as coronary and cerebrovascular arterial diseases. In other words, during the above period, such patients have to live not only with depressed work force but also under a reduced quality of life (QOL). On the other hand, the cause of atherosclerosis has been elucidated progressively; importance of known risk factors such as diabetes, hypertension and hyperlipemia have been confirmed. A treatment based on EBM for each disease has been established, and has been proved to be apparently effective. Therefore, when predictive markers indicative atherosclerosis have been found, tailor-made health care by applying appropriate medical treatment accordingly for each patients will be accomplished. Detection of incipient atherosclerosis by a laboratory testing, in particular, is considered very useful to prevent those complications.

In a pathologic process of atherosclerosis, an oxidized low density lipoprotein (LDL), in particular, is incorporated endlessly into cytoplasm of macrophages through scavenger receptors at the atherosclerotic lesion, and foam cells are accumulated in a typical incipient atherosclerotic lesion. Moreover, exacerbation of vasculitis grows by proliferation of vascular smooth muscle cells, transmigration of the cells toward lesion of atherosclerosis and transformation into a synthetic phenotype, formation of lipid core by necrosis of those foam cells, secretion of growth factors and inflammatory cytokines from activated macrophages and transformed vascular smooth muscle cells, and lesion of atherosclerosis becomes highly lipid-containing unstable plaques. Increased blood flow rate under vasoconstriction in hypertension causes shear stress of vessel wall, and it may lead to disruption or erosion of unstable plaques. Thereby, vascular subendothelial matrix will be exposed on blood vessel inner surface, resulting in acceleration of platelet aggregation and formation of intraarterial thrombus. As the results, blood supply to organs will be markedly reduced leading to the impaired function of those involved organs. The situation is serious in organs having the end artery, in which brain, heart and kidney are included.

According to "General Outline of Patients Survey" by the Ministry of Health, Labor and Welfare in Japan, annual number of patients with acute cardiac infarction is 110,000 (male: 79,000, female: 31,000), and patients with other ischemic heart diseases are 804,000 (male: 375,000, female: 429,000), and these numbers show increasing tendency year by year. Additionally, increased intake of saturated fatty acids by dietary habit of calorie-rich meal together with decreased exercise by prevalence of automobile, seem to predict increasing incidence of atherosclerotic complications. Further, incidence of diabetes which has been increasing reflected by such life style is one of the most influential risk factor of coronary arterial diseases. Based on the above background, it is predictable that population of patients with coronary arterial disease in Japan will increase to the same level as in the Western countries in the near future, threatening medical economy in Japan. Incidentally, health care cost for a patient with myocardial infarction in Japan is 3,500,000 yen in average. Prevention of outbreak of coronary arterial disease by 30 % can result in saving of total health care cost by about 115.5 billion yen annually.

To reduce risk of such diseases, urgent establishment of a simple and sensitive test procedure for detecting atherosclerosis is required in the field of health care.

On the other hand, it has been confirmed that Fcγ receptors are expressed on atherosclerotic lesion of arteries by the immunocytochemistry (Ratcliffe et al., Immunology letters 77,169-174,2001). Fcγ receptor (FcγRs) is a receptor for Fc region of IgG and classified to three subgroups, FcγRI, FcγRII and FcγRIII. Among these receptors, FcγRIII exists in two alternative forms, FcγRIIIa and FcγRIIIb. FcγRIIIa is an integral membrane glycoprotein expressed on natural killer (NK) cells, a subset of T lymphocytes, a subpopulation of monocytes and macrophages (Ravetch, J.V et al., J. Exp. Med., 170, 481-497, 1989). Difference between FcγRIIIa expressed on NK cells (hereafter abbreviated as FcγRIIIa^{NK}) and that on macrophages (FcγRIIIa expressed on macrophage, hereafter abbreviated as FcγRIIIa^{Mφ}) is considered in cell type-specific glycosylation pattern (de Haas, M. et aL, J. Immunol., 152, 900-907, 1994). In vitro experiments, FcγRIIIa^{Mφ} and FcγRIIIa^{NK} are confirmed to be released from cell surface as soluble form (soluble FcγRIIIa^{Mφ} derived form macrophage and soluble FcγRIIIa^{NK} derived from NK cell, hereinafter abbreviated as sFcγRIIIa^{Mφ} and sFcγRIIIa^{NK}, respectively) by activation of the cells (Harrison, D. et al., J. Immunol., 147, 3459-3465, 1991; Levy, P.C. et al., Am. J. Resp. Cell. Mol. Biol., 5, 307-314, 1991).

FcγRIIIb is a GPI (glycosyl-phosphatidyl-inositol)-anchored glycoprotein expressed exclusively on neutrophils, and it can be induced on eosinophiis (Huizinga, T.WJ. et aL, Nature, 333, 667-669, 1988). FcγRIIIb has two allotypes, NA1 and NA2, and released as soluble FcγRIIIb (hereafter abbreviated as sFcγRIIIb) from cell surface upon activation and during apoptosis of neutrophils (Huizinga, T.WJ. et al., Blood, 75, 213-217, 1990; Homburg, C.H.E. et aL, Blood, 85, 532-540,1995).

Although total soluble FcγRIII (hereafter abbreviated as total sFcγRIII) has been detected in saliva, synovial fluid, seminal fluid, serum and plasma (de Haas, M. et aL, J. Immunol., 152, 900-907, 1994; Huizinga, T.WJ. et aL, Blood, 75, 213-217, 1990; Huizinga, T.W.J. et al., Br. J. Haematol., 87, 459-463, 1994; Sautes, C. et al., Immunobiol., 185, 207-221, 1992; Galon, J. et al., Eur. J. Immunol., 28, 2101-2107, 1998; Koene, H.R. et al., Br. J. Haematol., 93, 235-241, 1996), none of the assays used discriminates sFcγRIIIa from sFcγRIIIb, or sFcγRIIIa^{Mφ} from sFcγRIIIa^{NK}. Plasma sFcγRIII is mainly FcγRIIIb derived from neutrophil and to a lesser extent sFcγRIIIa^{NK} derived from NK cell is (de Haas, M. et al., J. Immunol., 152, 900-907, 1994; Huizinga, TWJ. et al., Br. J. Haematol., 87, 459-463, 1994). sFcγRIIIa^{Mφ} derived from macrophage has not yet been detected in plasma

### DISCLOSURE OF THE INVENITON

The present invention has been accomplished in view of such circumstances as above, and an object of the present invention is to provide a novel method for determining a risks of atherosclerosis or future development of atherosclerotic complications on the basis of measuring an mount of sFcγRIIIa^{Mφ} in blood.

The present inventors have earnestly investigated to achieve the above-described purpose, and have obtained a monoclonal antibody specifically recognizing sFcγRIIIa^{Mφ}. Using this monoclonal antibody and another publicly known monoclonal antibody specifically recognizing FcγRIIIa, an amount (concentration) of sFcγRIIIa and sFcγRIIIa^{Mφ} in human plasma were measured and relations between the amounts and atherosclerosis was further investigated. As the results, sFcγRIIIa and sFcγRIIIa^{Mφ} were found to be predictive markers of atherosclerosis. Further, the present inventors found that it is able to not only diagnose atherosclerosis but also determine a risk of future development of atherosclerotic complications even for superficially healthy subjects by measuring an amount of sFcγRIIIa^{Mφ}, in particular, and the present invention has been accomplished.

That is, the present inventions consists of the following
(1) A method for determining a risk of atherosclerosis or future development of atherosclerotic complications, which comprises; measuring an amount of sFcγRIIIa (especially, FcγRIIIa^{Mφ}) in a blood sample from patients by using an antibody specifically recognizing FcγRIIIa (especially, FcγRIIIa^{Mφ}), and determining the risk on the basis of the result of the measurement.
(2) A method for determining a risk of atherosclerosis or future development of atherosclerotic complications, the method comprising the steps of; i) measuring an amount of total sFcγRIII in a blood sample from patients by using an antibody specifically recognizing FcγRIII, ii) measuring an amount of sFcγRIIIa^{Mφ} in a blood sample from patients by using an antibody specifically recognizing FcγRIIIa^{Mφ}, iii) calculating a ratio of the amount of sFcγRIIIa^{Mφ} to the amount of total sFcγRIII, and determining the risk on the basis of the result of calculation.
(3) A method for determining a risk of atherosclerosis or future development of atherosclerotic complications, the method comprising the steps of; i) measuring an amount of sFcγR IIIa in a blood sample from patients by using an antibody specifically recognizing FcγRIIIa, ii) measuring an amount of sFcγRIIIa^{Mφ} in a blood sample from patients is measured by using an antibody specifically recognizing FcγRIIIa^{Mφ}, and iii) calculating a ratio of the amount of sFcγRIIIa^{Mφ} to the amount of sFcγRIIIa, and determining the risk on the basis of the result of the calculation.
(4) A kit for measuring an amount of sFcγRIIIa^{Mφ}, which comprises a solid phase coated with an antibody specifically recognizing FcγRIIIa^{Mφ}, and a labeled antibody specifically recognizing FcγRIII or FcγRIIIa.
(5) A kit for measuring an amount of sFcγRIIIa^{Mφ} and an amount of total sFcγRIII, which comprises a solid phase coated with an antibody specifically recognizing FcγRIIIa^{Mφ}, a labeled antibody specifically recognizing FcγRIII or FcγRIIIa, a solid phase coated with an antibody specifically recognizing FcγRIII, and a labeled antibody specifically recognizing FcγRIII.
(6) A kit for measuring an amount of sFcγRIIIa^{Mφ} and an amount of sFcγRIIIa, which comprises a solid phase coated with an antibody specifically recognizing FcγRIIIa^{Mφ}, a labeled antibody specifically recognizing FcγRIII or FcγRIIIa, and a solid phase coated with an antibody specifically recognizing sFcγRIIIa.
(7) A kit for determining a risk of atherosclerosis or future development of atherosclerotic complications, which comprises a solid phase coated with an antibody specifically recognizing FcγRIIIa^{Mφ}, and a labeled antibody specifically recognizing FcγRIII or FcγRIIIa.
(8) A kit for determining a risk of atherosclerosis or future development of atherosclerotic complications, which comprises a solid phase coated with an antibody specifically recognizing FcγRIIIa^{Mφ}, a labeled antibody specifically recognizing FcγRIII or FcγRIIIa, and a solid phase coated with an antibody specifically recognizing FcγRIII.
(9) A kit for determining a risk of atherosclerosis or future development of atherosclerotic complications, which comprises a solid phase coated with an antibody specifically recognizing FcγRIIIa^{Mφ}, a labeled antibody specifically recognizing FcγRIII or FcγRIIIa, and a solid phase coated with an antibody specifically recognizing FcγRIIIa.

### BRIEF DESCRIPTION OFTHE DRAWINGS

Fig.1 shows a calibration curve used in Example 1 to measure an amount of total sFcγRIII by ELISA.
Fig.2 shows a calibration curve used in Example 1 to measure an amount of sFcγRIII^{Mφ} by Immuno-PCR.
Fig.3(a) shows the amount of sFcγRIIIa^{Mφ} in plasma from healthy subjects and patients with coronary arterial disease (CAD), obtained in Example 1.
Fig.3(b) shows the amount of sFcγRIIIa in plasma from healthy subjects and patients with CAD, obtained in Example 1.
Fig.3(c) shows the amount of total sFcγRIII in plasma from healthy subjects and patients with CAD, obtained in Example 1.
Fig.4 shows the amount of sFcγRIIIa^{Mφ} in plasma from patients with CAD and severity of CAD, obtained in Example 2.
Fig.5 shows a correlation between the amount of sFcγRIIIa^{Mφ} in plasma and ages in healthy subjects obtained in Example 3.
Fig.6 shows the ratio of sFcγRIIIa^{Mφ}/total sFcγRIII in plasma from healthy subjects and patients with CAD, obtained in Example 4.
Fig.7 shows the ratio of sFcγRIIIa^{Mφ}/sFcγRIIIa in plasma from healthy subjects and patients, obtained in Example 5.
Fig.8 shows the amount of sFcγRIIIa^{Mφ} in plasma from healthy subjects and patients with CAD obtained in Example 6.
Fig.9 shows the amount of total sFcγRIII in plasma from healthy subjects and patients with CAD, obtained in Example 6.
Fig.10 shows the ratio of sFcγRIIIa^{Mφ}/total sFcγRIII in plasma from healthy subjects and patients with CAD, obtained in Example 6.

### BEST MODE FOR CARRYING OUT THE INVENTION

In the present invention, an antibody specifically recognizing FcγRIIIa^{Mφ} (hereafter abbreviated as anti-FcγRIIIa^{Mφ} antibody) used for measuring an amount (concentration) of sFcγRIIIa^{Mφ} is an antibody reactive with sFcγRIIIa^{Mφ} (that is, recognizes sFcγRIIIa^{Mφ}), and substantially is not reactive with sFcγRI, sFcγRII, sFcγRIIIb, sFcγRIIIa^{NK} (that is, does not recognize sFcγRI, sFcγRII, sFcγRIIIb, sFcγRIIIa^{NK}).

In the present invention, an antibody specifically recognizing FcγRIIIa (hereinafter abbreviated as anti-FcγRIIIa antibody) used for measuring an amount of sFcγRIIIa is an antibody reactive with sFcγRIIIa (sFcγRIIIa^{Mφ} and sFcγRIIIa^{NK}) (that is, recognizes sFcγRIIIa^{Mφ} and sFcγRIIIa^{NK}), and substantially is not reactive with sFcγRI, sFcγRII and the like (that is, does not recognize sFcγRI, sFcγRII and the like).

Also, an antibody specifically recognizing FcγRIII (hereinafter abbreviated as anti-FcγRIII antibody) used in the present invention is reactive with sFcγRIII (sFcγRIIIa and sFcγRIIIb) (that is, recognizes sFcγRIIIa and sFcγRIIIb), and substantially is not reactive with sFcγRI and sFcγRII (that is, does not recognize sFcγRI and sFcγRII).

Origins of these antibodies are not limited, and either polyclonal antibody or monoclonal antibody can be used. Also, target-specific bonding molecules having appropriate properties such as a phage antibody or an aptamer prepared by molecular evolutionary engineering can also be used as alternatives to the above-described antibodies. They can be optionally used in singly or in a proper combination. However, in view of the specificity of an antibody having uniform properties, a monoclonal antibody is preferable compared to a polyclonal antibody.

A method for preparing a polyclonal antibody used in the present invention includes a conventional procedure, wherein an animal such as horse, cattle, sheep, rabbit, goat, guinea pig, rat or mouse is immunized with a substance to be measured, and the method is described in, for example, Tadashi Matsuhashi et al., Men-ekigaku Jikken Nyumon, 2^{nd} ed., Gakkai-Shuppan Center Ltd., 1981.

A method for preparing the monoclonal antibody used in the present invention includes a procedure comprising, hybridizing immunologically sensitized cells such as spleen cells or lymphocytes from an animal including rat or mouse immunized with a substance to be measured with permanently growing cells, such as myeloma cells to make hybridoma according to a publicly known cell fusion method such as a method established by Köhler and Milstein (Nature, 256, 495, 1975), selecting a hybridoma which produces a specific antibody against the substance to be measured, culturing said hybridoma in a medium or inoculating the cell by intraperitonial injection of a host animal to produce the antibody in peritoneal fluid, and isolating an objective monoclonal antibody from the corresponding medium or peritoneal fluid. The other method also includes a publicly known method applying such as recombinant DNA technology (Eur. J. Immunol., 6, 511, 1976), and then culturing these cells to obtain the objective monoclonal antibody.

Commercially available antibodies can be used, and if necessary, these antibodies can be used after being digested with an enzyme such as pepsin or papain into F(ab')₂, Fab' or Fab.

In addition, an aptamer can be prepared by a method described in U.S.P. No.270,153.

A monoclonal antibody used in the present invention can be obtained by applying a conventional procedure. For example, an anti-FcγRIIIa^{Mφ} antibody and hybridoma producing the same can be obtained by applying a conventional procedure using FcγRIIIa^{Mφ} as an immunizing antigen.

Briefly, FcγRIIIa^{Mφ} used as an immunizing antigen can be prepared, for example, by lysing cultured monocytes from citrated human blood using Percoll density centrifugation and subsequent counterflow centrifugal elutriation. The protein can be purified from the lysate by publicly known methods, for example, affinity chromatography using Sepharose beads coated with an anti-FcγRIII antibody or an anti-FcγRIIIa antibody, or in combination thereof or with other several chromatographic techniques.

A method for obtaining an anti-FcγRIIIa^{Mφ} antibody of the present invention using FcγRIIIa^{Mφ} obtained by the above-described method is further described in more detail bellow.

Briefly, FcγRIIIa^{Mφ} obtained by the above-described method is mixed with adjuvant such as incomplete Freund's adjuvant to make a suspension. The above-described suitable animal is immunized by inoculation of the suspension through subcutaneously, intravenously or intraperitoneally with an appropriate dosage, for example, generally 0.1 to 100 µg per inoculation, preferably 0.1 to 10 µg as the amount of FcγRIIIa^{Mφ} per inoculation, generally 3 to 10 times, preferably 3 to 8 times, in every 1 to 5 weeks, preferably every 2 to 5 weeks, After immunization, a blood sample is obtained from the animal and the serum is examined for its reactivity with FcγRIIIa^{Mφ} by a publicly known method, for example, solid phase enzyme -linked immunosorbent assay (ELISA) using a solid phase coated with FcγRIIIa^{Mφ}. After the reactivity is completed the spleen is taken out from the immunized animal at 3 to 4 days after the final immunization, and spleen cells are prepared by a publicly known method. The spleen cells obtained are hybridized with myeloma cell line such as NS-1, Sp2 and X63 by a conventional method to be subjected to HAT selection by a publicly known method. Thus selected fused cells are cultured, and the supernatant of each culture medium is subjected to conventional methods such as ELISA, indirect immunofluorescence technique, or Western blotting immunostaining on polyvinylidendifluoride (PVDF) membrane after SDS-poiyacxylamide gel electrophoresis for further selection of anti-FcγRIIIa^{Mφ} antibody producing hybridoma with the above-described properties. Then, the cell, which is confirmed to produce an antibody stably with high titer by repeated end-point titration, is finally selected as an anti-FcγRIIIa^{Mφ} antibody producing hybridoma cell line.

Then, the hybridoma cell obtained above is inoculated by intraperitoneal injection to a host animal to produce an anti-FcγRIIIa^{Mφ} antibody in the ascites. The ascites is withdrawn from the animal, and the anti-FcγRIIIa^{Mφ} antibody is purified according to a method commonly employed in this field, for examples, salting-out by ammonium sulfate, dialysis against a buffered solution such as phosphate buffer, DEAE-cellulose chromatography and FcγRIIIa^{Mφ} affinity chromatography to obtain a purified anti-FcγRIIIa^{Mφ} anti-body.

The subclass of thus obtained monoclonal antibody can be determined by publicly known methods such as a double immunodiffusion method [Rinsho-kensahou Teiyou, ver. 30, Kinbara Shuppan Co., Ltd., p.842-843].

A typical example of an anti-FcγRIII antibody used in the present invention includes, for example, CLBFcRgranI, CLB-LM6.30 and GRM1 (de Haas, M. et aL, Leukocyte typing V, S.F. Schlossmann et al. eds. Oxford University Press, 811-817, 1995). In this case, GRM1 recognizes FcγRIIIa and NA2-FcγRIIIb, but not reactive with NA1-FcγRIIIb.

An anti-FcγRIIIa^{Mφ} antibody includes monoclonal antibody MKGR14 produced by hybridoma cell line MKGR14. The hybridoma MKGR14 has been deposited to International Patent Organism Depositary of National Institute of Advanced Industrial Science and Technology (AIST Tsukuba Central 6,1-1, Higashi 1-Chome Tsukuba-Shi, Ibaraki-ken, 305-8566, Japan) as of January 29, 2002 with a deposition number of FERM BP-7866.

Amounts of total sFcγRIII, sFcγRIIIa or sFcγRIIIa^{Mφ} in blood sample in the present invention can be measured with a publicly known immunological method such as enzyme linked immunosorbent assay (ELISA) or radio immunoassay (RIA). However, as amounts of sFcγRIIIa and sFcγRIIIa^{Mφ} in plasma of healthy subject is very low, it is preferable to measure these amounts by using a high sensitive assay method such as chemiluminescence immunoassay (CLEIA) or immuno-PCR (immuno-polymerase chain reaction) (Furuya, D. et aL, J. Immunol. Methods, 238, 173-180,2000).

In one aspect of the present invention, an method for measuring an amount of total sFcγRIII or sFcγRIIIa by ELISA is described bellow, taking the method for measuring an amount of total FcγRIIIa as an example.

Briefly, a blood sample incubated with a solid phase which is coated with anti-FcγRIII antibody (a primary antibody) and reacted at 4 to 40°C for 0.5 to 16 hours to form an antigen-antibody complex (an coated anti-FcγRIII antibody-sFcγRIII complex) on the solid phase. Further, the solid phase is reacted with labeled anti-FcγRIII antibody (a secondary antibody) at 4 to 40°C for 0.5 to 16 hours to form a labeled antigen-antibody complex (an coated anti-FcγRIII anh'body-sFcyRIII-labeled anti-FcyRIII antibody complex), and then an amount of labeling substance in the labeled antigen-antibody complex on the solid phase is measured. The obtained value is applied to a calibration curve showing the relationship between an amount of labeling substance (measured value) and an amount of total sFcyRII), which is previously obtained by the similar method as described above using known amounts of total sFcγRIII solutions and the same reagents as above. Thus the amount of total sFcγRIII in a sample can be determined. Otherwise, an appropriate standard (for example, pooled plasma of normal healthy subjects) is determined as 100 AU (arbitrary units). The amount of total sFcγRIII can be obtained as a relative value to the appropriate standard.

In another aspect of the present invention, when a labeled antibody (a secondary antibody) is a biotin-labeled antibody, streptavidin which has been labeled with an enzyme such as peroxidase is reacted with a biotin-labeled andgen-antibody complex to form an enzyme labeled streptavidin-biotin labeled antigen-antibody complex on the surface of corresponding solid phase, followed by reacting labeled enzyme with substrate thereof to measure an amount of the enzyme in the enzyme labeled streptavidin-biotin labeled antibody-antigen complex on the standard solid phase. By a similar method to the above-described, an amount of total sFcγRIII in a sample can be determined from the calibration curve which previously prepared.

A measurement procedure of amounts of total sFcγRIII, sFcγRIIIa or sFcγRIIIa^{Mφ} by ELISA using a biotin-labeled secondary antibody of the present invention is outlined bellow, taking an method for measuring an amount of total FcγRIII as an example.

Briefly, blood sample from patients are incubated with a solid phase which is coated with an anti-FcγRIII antibody (a primary antibody) to form an antigen-antibody complex on the solid phase. Then, the solid phase is reacted with a biotin-labeled rabbit anti-FcγRIII antibody (secondary antibody) to form a biotin-labeled antigen-antibody complex, followed by reacting with peroxidase-labeled streptavidin to form a peroxidase-labeled antigen-antibody complex, and then reacting with substrates as tetramethylbenzidine and hydrogen peroxide (H₂O₂) to measure an amount of labeling substance in the labeled antigen-antibody complex on the solid phase. The obtained value is applied to a calibration curve showing the relationship between an amount of labeling substance and an amount of total sFcγRIII, which is previously obtained by similar method as described above using known amounts s of total sFcγRIII solutions and the same reagents as above. Thus an amount of total sFcγRIII in a sample can be determined.

A measurement procedure of an amount of sFcγRIIIa^{Mφ} by ELISA, for example, can be performed as following procedure.

Briefly, a blood sample is incubated with a solid phase which is coated with anti-FcγRIIIa^{Mφ} antibody (a primary antibody) and reacted at 4 to 40°C for 0.5 to 16 hours to form an antigen-antibody complex (an coated anti-FcγRIIIa^{Mφ} antibody-sFcγRIIIa^{Mφ} complex) on the solid phase. Further the solid phase is reacted with a labeled anti-FcγRIII antibody or a labeled anti-FcγRIIIa antibody (a secondary antibody) at 4 to 40°C for 0.5 to 16 hours to form a labeled antigen-antibody complex (an coated anti-FcγRIIIa^{Mφ} antibody-sFcγRIIIa^{Mφ} labeled antibody complex), and an amount of labeling substance in the labeled antigen-antibody complex on the solid phase is measured. The obtained value is applied to a calibration curve showing the relationship between an amount of labeling substance (measured value) and an amount of sFcγRIIIa^{Mφ}, which is previously obtained by a similar method as described above using known amounts of sFcγRIIIa^{Mφ} solutions and the same reagents as above. An amount of the sFcγRIIIa^{Mφ} in a sample can be determined.

When a labeled antibody (a secondary antibody) is a biotin-labeled antibody, the measurement can be performed by a similar method as in measurement of an amount of total sFcγRIII using a biotin-labeled antibody.

An amount of sFcγRIIIa^{Mφ} can also be measured similarly by contrast, using an anti-FcγRIII antibody or an anti-FcγRIIIa antibody as a primary antibody coated on a solid phase, and a labeled anti-FcγRIIIa^{Mφ} antibody as a secondary antibody.

Also, an amount of sFcγRIIIa can be measured by an ELISA method according to the above-described procedure.

Additionally, the above-described examples of measurement methods of an amount of total sFcγRIII, sFcγRIIIa or sFcγRIIIa^{Mφ} by ELISA are based on principle of non-competing assay using an antibody coated solid phase, so called a sandwich method. However, it is needless to say that the measurement method of the present invention can be performed using free primary and secondary antibodies without using a solid phase.

A solid phase used in the above method can be any kind as long as it is commonly used for an immunological assay method such as ELISA, and includes preferably a synthetic polymer such as polystyrene, polypropylene, polyacrylic acid, polymethacrylic acid, polyacrylamide, polyglycidyl methacrylate, polyvinyl chloride, polyethylene, polychlorocarbonate, a silicon resin, silicon rubber; and inorganic substances such as porous glass, ground glass, alumina, silica gel, activated carbon and metal oxide. These solid phases can be used in wide variety of shape such as tubes, beads, disks, micro particles (latex particles) and micro plates. Above all, micro plates are preferable, in particular, from the points of easy washing and easy handling on simultaneous treatment of many samples.

As to an immobilizing method of the above-described antibodies on these solid phases, known immobilization methods such as immobilization by chemical binding methods or physical adsorption methods (Japanese Patent Publication (KOKOKU) No. (Hei) 5-41946/1993) can be applied.

A specific example of the immobilizing method includes contacting solutions of an ant-FcγRIII antibody, anti-FcγRIIIa antibody or anti-FcγRIIIa^{Mφ} antibody in an amount of generally 0.1 µg/mL to 20 mg/mL, preferably 1 µg/mL to 5 mg/mL with a solid phase, followed by keeping them at appropriate temperature for predetermined period to obtain an antibody coated solid phase.

A solvent used to prepare solutions of an ant-FcγRIII antibody, an anti-FcγRIIIa antibody or an anti-FcγRIIIa^{Mφ} antibody can be any kind unless it interferes adsorption or binding of said antibody of interest onto the solid phase and preferably includes, for example, purified water and a buffer solution such as phosphate buffer, Tris buffer, Good's buffer, glycine buffer and borate buffer with pH 5.0 to 10.0, preferably pH 6.5 to 85. An amount of a buffering agent in these buffer solutions is selected as appropriate generally from 10 to 500 mM and preferably from 10 to 300 mM. This solution may contain substances such as sugar, salts such as sodium chloride (NaCI), detergents, preservatives and proteins, in the amount not to interfere the adsorption or binding of an antibody of interest onto the solid phase.

Blocking treatment is commonly conducted in this field, that is, further dipping of thus obtained antibody bound solid phase in a solution of unrelated protein to the antibody, such as bovine serum albumin, milk protein including skimmed milk, and ovalbumin, is preferable to prevent nonspecific reaction on measurement.

In the present invention, a labeling substance used for labeling an ant-FcγRIII antibody, anti-FcγRIIIa antibody or an anti-FcγRIIIa^{Mφ} antibody which is used for measuring an amount of total sFcγRIII, sFcγRIIIa or sFcγRIIIa^{Mφ} includes all of labeling substances commonly used in this field, including for example, alkali phosphatase, β-galactosidase, peroxidase, microperoxidase, glucoseoxidase, glucose-6-phosphate dehydrogenase, acetylcholinesterase, malic dehydrogenase, luciferase and other enzymes, which are used in EIA, ^{99m}Tc, ¹³¹I, ¹²⁵I, ¹⁴C, ³H and other radioisotopes, which are used in RIA, fluorescence, dansyl, fluorescamine, coumarin, naphthylamine and their derivatives and other fluorescent substances, which are used in FIA, luciferin, isoluminol, luminol, bis (2,4,6-trifluorophenyl) oxalate and other luminescent substances, phenol, naphthol, anthracene and their derivatives and other substances, which can absorb an ultraviolet light, 4-amino-2,2,6,6-tetramethylpiperidine-1-oxyl, 3-amino-2,2,5,5-tetramethylpyrrolidine-1-oxyl, 2,6-di-t-butyl-α-(3,5-di-t-butyl-4-oxo-2,5-cyclohexadien-1-ylidene)-p-tolyl oxyl and other oxyl group-containing compounds, which have characteristics as spin-labeling agent.

The method for binding (labeling) the above labeling substance to an antibody can be conducted after a per se known manner for binding a labeling substance with an antibody generally used in publicly known EIA, RIA or FIA (e.g. Yuichi Yamamura "Ikagaku Jikken Koza" Vol.8, 1st ed., NAKAYAMA-SHOTEN Ltd., 1971; Akira Kawano "Zusetsu Keikokotai" 1st ed, Soft Science, Inc., 1983; and Eiji Ishikawa, Tadashi Kawai and Kiyoshi Miyai "Koso Men-eki Sokuteiho" 2nd ed., IGAKU-SHOIN Ltd., 1982). As the labeling method, a conventional one using a reaction of avidin (or streptavidin) with biotin can be used.

In application of a reaction between avidin (or streptavidin) and biotin, preferable method for attaching biotin to an antibody is using a commercially available biotinylation reagents, more specifically, such as biotin having a succinimide group introduced thereinto (for example, NHS-biotin) or a product obtained by combing N-hydroxysuccinimide (NHS) and biotin through a spacer with the amino group of an antibody or an antigen (see, for example, J. BioL Chem., 264, 272-279,1989); a method of reacting, for example, a commercially available N-[6-(Biotinamide) hexyl]-3'-(2'-pyridyldithio) propionamide (biotin-HPDP) or N-Iodoacetyl-N-biotinylhexylenediamin with the thiol group of an antigen or an antibody (see, for example, Ann. New York Acad. Sci., 254, 203, 1975); and a method of reacting biotin having a hydrazino group introduced thereinto with the aldehyde group of an aldehyde-modified antigen or an antibody (see, for example, J. BioL Chem., 172, 71, 1948; Biotech. AppL Biochem., 9, 488-496, 1987).

As to the degree of the biotination of an antigen or an anhbody, the amount of the compound is about 0.2 to 10 moles, preferably about 1 to 5 moles, per mole of the antigen or antibody When the degree of the biotination is too high, there is a problem, for example, in increased insolubility of the antigen or antibody or inhibited antigen-antibody reaction. When the degree of biotination is too low, there is a problem, for example, in low the sensitivity. Therefore, care should be taken in the modification.

Commercially available enzyme-labeled avidin or streptavidin can be used as it is without specific limitation on quality and purity. Although the concentration of avidin is somewhat varied depending on the quantity of labeled biotin for a antigen (or an antibody) against a substrate to be measured or on the assay items, and is not specifically limited, the concentration in the reaction solution can be properly chosen usually in the range of 0.01 to 5000 µg/L, preferably 0.1 to 1000 µg/L and more preferably 5 to 1000 µg/L.

Additionally, this solution may contain substances commonly used in this field as stabilizers such as sugar, proteins and surfactants in the range of an amount commonly used in this field.

An measurement method of labeling substance in a primary antibody-antigen-secondary antibody complex formed by an antigen-antibody reaction depends on types of labeling substances, however, the measurement can be performed based on property detectable by any of methods in each labeling substance. For example, when the property is enzyme activity, the measurement is carried out according to a conventional method of EIA, for example, the method described in Tsunehiro Kitagawa, Toshio Nanbara, Akio Tsuji, and Eiji Ishikawa "Koso Men-eki Sokuteiho," an extra issue No.31 of Tanpakushitsu Kakusan Koso, pp.51-63, KYORITSU-SHUPEAN Ltd., published on Sep.10, 1987 etc. When the detectable substance is a radioisotope, the measurement is carried out according to a conventional method of RIA by property choosing and using a measuring instrument such as Geiger-Müller (GM) counter, a liquid scintillation counter, a well-type scintillation counter, an HPLC counter or the like depending on the kind and intensity of radiation ray emitted by said radioisotope (see, for example, Yuichi Yamamura, "Ikagaku Jikken Koza," vol.8, 1^{st} ed., NAKAYAMA-SHOTEN Ltd., 1971). When said property is fluorescence-emitting properties, the measurement is carried out according to a conventional method of FIA using a measuring instrument such as a fluorophotometer described, for example, the method described in Akira Kawano, "Zusetsu Keikokotai," 1^{st} ed., Soft Science Inc., 1983 etc. When the property is luminescence-emitting properties, the measurement is carried out according to a conventional method using a measuring instrument such as a photo counter described, for example, the method described in Tsunehiro Kitagawa, Toshio Nanbara, Akio Tsuji, and Eiji Ishikawa, ''Koso Men-eki Sokuteiho," an extra issue No.31 of Tanpakushitsu Kakusan Koso, pp.252-263, KYORITSU-SHIUPEAN Ltd., published on Sep.10, 1987, etc. When the property is ultraviolet-light absorbing properties, the measurement is carried out by a conventional method using a measuring instrument such as a spectrophotometer. When the detectable substance is a substance having properties as spin, the measurement is carried out according to a conventional method using an electron spin resonance apparatus described, for example, the method described in Tsunehiro Kitagawa; Toshio Nanbara, Akio Tsuji, and Eiji Ishikawa, "Koso Men-eki Sokuteiho," an extra issue No.31, of Tanpakushitsu Kakusan Koso, pp.264-271, KYORITSU-SHUPPAN Ltd., published on Sep.10, 1987, etc.

For more specifically, when a labeling substance is enzyme, an assay method includes a publicly known method such as a reaction with coloring reagent and subsequent measurement of an amount of dye generated from the reaction, using a spectrophotometer,etc.

A coloring reagent used for such purpose is a conventionally used coloring agent in this field, including for example, tetramethylbenzidine, o-phenylenediamine, o-nitrophenyl-β-D-galactoside, 2,2'-azino-bis(3-ethylbenzthiazoline-6-sulfonic acid) (ABTS), N-ethyl-N-sulfopropyl-m-anisidine (ADPS) and p-nitrophenylphosphoric acid.

To terminate a coloring reaction, a commonly used termination method, for example, addition of an enzyme inhibitor such as 1 to 6N sulfuric acid can be applied.

A blood sample used in the present invention includes blood, serum, plasma, etc. However, as sFcγRIIIa^{Mφ} and the like are released from each of the relevant leukocyte, for example, upon activation, EDTA-added plasma is preferable because of its less influence by post blood-drawing. An amount of total sFcγRIII in EDTA-added plasma has been reported not to be influenced by incubating at room temperature and a repeated freeze-thaw treatment (Koene, H.R. et al., Br. J. Haematol., 93, 235-241, 1996).

Measurement of amounts of sFcγRIIIa and sFcγRIIIa^{Mφ} by an immuno-PCR method can be performed according to a method reported by Furuya et aL (Furuya, D. et aL, J. ImmunoL Methods, 238, 173-180, 2000), and the measurement procedure will follow in more practically in the case of the latter measuring method of an amount of sFcγRIIIa^{Mφ}.

First, using an appropriate template such as plasmid Bluescript (2.96 kb from Toyobo Co., Ltd.) and, for example, a biotin-labeled primer and an unlabeled reverse primer, polymerase chain reaction (PCR) amplification is carried out. The PCR product obtained is further purified to prepare biotinylated DNA.

A blood sample is then added to a PCR plate previously coated with an anti-FcγRIIIa^{Mφ} antibody (a primary antibody) to form an antigen-antibody complex, followed by a reaction with a biotin-labeled anti-FcγRIII antibody (a secondary antibody) to form a biotin-labeled antigen-antibody complex, reacting with avidin to form an avidin-labeled antigen-antibody complex, further reacting with the above-prepared biotinylated DNA to form a DNA-labeled antigen-antibody complex and amplificating the labeled DNA by PCR using appropriate combination of primers. The PCR product obtained above is separated and measured by agarose gel electrophoresis, and based on whose measurement value, an amount of sFcγRIIIa^{Mφ} in a sample can be determined. An amount of the labeled DNA in each well can also be measured by a real time PCR method using, for example, an ABI PRISM 7700 Sequence Detection System (from Applied Biosystems Japan Inc.).

As a template used for an immuno-PCR method, various types of vectors commonly used in this field can be used, and as a primer and a biotin-labeled primer commonly used in this field can also be used, and those obtained on the market may also be used. Equipments commonly used in this field may also be used. Additionally, for denaturing in PCR procedure, temperatures and times of annealing and elongation reaction can be optimized according to combinations of primers used.

Other reagents and measuring conditions (reaction temperature, reaction time, wave length of measurement, measuring instrument and so on) in practicing measuring method of the present invention can be selected according to the above-described publicly known immunological assay method, and the assay can be performed according to a publicly known immunological assay method, and any instrument, such as an automatic analyzer and a spectrophotometer commonly used in this field can be used without exception.

Specific examples of buffer solutions usable in the present invention include all known buffers commonly used as assay methods based on an antigen-antibody reaction, for example, solutions of Tris buffer, phosphate buffer, Veronal buffer, borate buffer, Good's buffer, and pH range thereof is not limited as long as an antigen-antibody reaction is not inhibited. Usually, the pH is preferably chosen in the range of 5 to 9.

Term "pre-determined amount" used in the method for determining a risk of atherosclerosis or future development of atherosclerotic complications of the present invention includes an amount of sFcγRIIIa, especially an amount of sFcγRIIIa^{Mφ} in a sample originated from blood of a healthy subject, or a ratio of an amount of sFcγRIIIa^{Mφ} to an amount of total sFcγRIII, or a ratio of an amount of sFcγRIIIa^{Mφ} to an amount of sFcγRIIIa in a blood sample from healthy subjects.

The method for determining a risk of atherosclerosis or future development of atherosclerotic complications of the present invention includes a method comprising, at first an amount of sFcγRIIIa (preferably sFcγRIIIa^{Mφ}) in a blood sample from a healthy subject is measured by the measurement method of the present invention. Using the thus obtained amount of sFcγRIIIa as a reference value, an amount of sFcγRIIIa (preferably sFcγRIIIa^{Mφ}) in a sample to be diagnosed is measured by using the similar procedure, and then, the amount is compared with the standard value. When the value is significantly higher than the standard value, it is diagnosed that the sample is in high risk of atherosclerosis or in high risk of the future development of atherosclerotic complications.

Also, an amount of sFcγRIIIa (preferably sFcγRIIIa^{Mφ}) in a blood sample from a healthy subject is measured by the measurement method of the present invention, and the thus obtained amount of sFcγRIIIa is used as a standard reference (100 arbitrary units (AU)). Separately, an amount of sFcγRIIIa (preferably sFcγRIIIa^{Mφ}) in a sample to be diagnosed is measured by using the similar procedure, and a ratio of the amount to the standard reference is calculated. When the value (ratio) is significantly higher than the standard reference, it is diagnosed that the sample is in high risk of atherosclerosis and in high risk of the future development of atherosclerotic complications.

Further, the following method for determining a risk of atherosclerosis or future development of atherosclerotic complications can be used, that is, an amount of sFcγRIIIa^{Mφ} and an amount of total sFcγRIII or sFcγRIIIa in a blood sample from a healthy subject is measured by the method of the present invention, and a ratio of the amount of sFcγRIIIa^{Mφ} to the amount of total sFcγRIII or a ratio of the amount of sFcγRIIIa^{Mφ} to the amount of sFcγRIIIa is calculated. On the other hand, an amount of sFcγRIIIa^{Mφ} and an amount of sFcγRIII or an amount of total sFcγRIIIa in a sample to be diagnosed are measured, and a ratio of the amount of sFcγRIIIa^{Mφ} to the amount of total sFcγRIII or a ratio of the amount of sFcγRIIIa^{Mφ} to the amount of sFcγRIIIa is calculated. When the value (ratio) is significantly higher than the value (ratio) which is obtained by using a blood sample from a healthy subject, it is diagnosed that the sample is in high risk of atherosclerosis or in high risk of the future development of atherosclerotic complications.

A method using a ratio mentioned above is more preferable, because influences affecting the value (ratio) are minimized, even if it is diabetes-originated atherosclerosis with the particular development process. Among methods using ratio, a method using a ratio of an amount of sFcγRIIIa^{Mφ} to an amount of total sFcγRIII is more preferable.

Whether the present condition is in high risk of atherosclerosis or in high risk of developing atherosclerotic complications in the future can be determined by measuring an amount of sFcγRIIIa (preferably sFcγRIIIa^{Mφ}) only, but it can be determined based on the above-described ratio. In this case, measured values of labeling substances derived from a labeled anti-FcγRIII antibody, a labeled anti-FcγRIIIa antibody and a labeled anti-FcγRIIIa^{Mφ} antibody are proportional to the amount of total sFcγRIII, sFcγRIIIa and sFcγRIIIa^{Mφ}, respectively, and thus, absolute amount is not necessary needed but the ratio can be determined using measured values of the labeling substances.

The kits for measuring an amount of sFcγRIIIa^{Mφ} and total sFcγRIII or FcγRIIIa of the present invention comprise, at least as described previously, (1) a kit comprising a solid phase coated with an anti-FcγRIIIa^{Mφ} antibody and a labeled anti-FcγRIII antibody or a labeled anti-FcγRIIIa antibody, (2) a kit comprising a solid phase coated with an anti-FcγRIIIa^{Mφ} antibody, a labeled anti-FcγRIII antibody or a labeled anti-FcγRIIIa antibody, and a solid phase coated with an anti-FcγRIII antibody and a labeled anti-FcγRIII antibody or (3) a kit comprising a solid phase coated with a anti-FcγRIIIa^{Mφ} antibody, a labeled anti-FcγRIII antibody or a labeled anti-FcγRIIIa antibody, and a solid phase coated with a anti-FcγRIIIa antibody, and preferred embodiment of constitutive elements and practical examples are as described above. Additionally, the above kit (2) is used for calculating a ratio of an amount of sFcγRIIIa^{Mφ} to an amount of total sFcγRIII, and the kit (3) is used for calculating a ratio of an amount of sFcγRIIIa^{Mφ} to an amount of sFcγRIIIa.

The kits of the present invention for determining a risk of development of atherosclerosis or future development of atherosclerotic complications of the present invention comprise, at least as described previously, (1) a kit comprising a solid phase coated with an anti-FcγRIIIa^{Mφ} antibody and a labeled anti-FcγRIII antibody or a labeled anti-FcγRIIIa antibody, (2) a kit comprising a solid phase coated with an anti-FcγRIIIa^{Mφ} antibody, a labeled anti-FcγRIII antibody or a labeled anti-FcγRIIIa antibody, and a solid phase coated with an anti-FcγRIII antibody and a labeled anti-FcγRIII antibody, or (3) a kit comprising a solid phase coated with an anti-FcγRIIIa^{Mφ} antibody, a labeled anti-FcγRIII antibody or a labeled anti-FcγRIIIa antibody, and a solid phase coated with an anti-FcγRIIIa antibody, and preferred embodiment of constitutive elements and practical examples are as described above. Additionally, the above kit (2) is used for calculating a ratio of an amount of sFcγRIIIa^{Mφ} to an amount of total sFcγRIII, and kit (3) is used for calculating a ratio of an amount of sFcγRIIIa^{Mφ} to an amount of sFcγRIIIa, and then, determining a risk of atherosclerosis or future development of atherosclerotic complications on the basis of the results of calculation.

In the following, the present invention is further explained in detail, and the present invention is not limited thereto by any means.

### EXAMPLES

Plasmas from patients with CAD and plasma from healthy subjects used in the following Reference Examples and Examples 1 to 5, and an anti-FcyRIII antibody used in the following Reference Examples and Examples 1 to 6 are shown as follows, and statistical data analysis methods are also shown bellow.

### (Plasma)

Plasmas of patients with coronary artery disease (hereinafter designated as CAD) were obtained from patients with NA (1+, 2-) phenotype (n = 45, 63.0 ± 10.0 years old), selected from patients hospitalized at the Division of Cardiology, Kansai Medical University Hospital from May 1999 to February 2000. All patients met The American Heart Association Criteria for CAD. None of all patients with CAD had any evidence of renal, hepatic, infectious, inflammatory disease or diabetes mellitus.

Plasmas from healthy subjects for NA phenotype were obtained from 107 healthy volunteers with NA(1+, 2-) phenotype (19 to 75 years old, 42.9 ± 14.3 years old) randomly recruited from the hospital staffs. None of these healthy subjects had any evidence of renal, hepatic, hyperlipemia, infectious disease, inflammatory disease and diabetes mellitus and none were taking any medication. These plasmas from healthy subjects were also used as pooled plasma for setting 100 AU for measuring an amount of sFcγRIIIa^{Mφ}, etc. Among them, 27 age matched individuals were selected as healthy controls. Clinical characteristics of both groups are shown in Table 1.

**Table 1**

| | Control | Patient with CAD |
|---|---|---|
| n | 27 | 45 |
| Age | 60.5±8.6 | 633±103 |
| BMI (kg/m²) | 22.9±2.8 | 24.4±3.5 |
| Systolic pressure (mmHg) | 121±15 | 129±21 |
| Diastolic pressure (mmHg) | 75±11 | 75±13 |
| Number (%) of cases of hypertension | - | 18(40) |
| Number (%) of cases of hyperlipidemia | - | 13(29) |

### (an anti-FcγRIII antibody)

Anti-FcγRIII monoclonal antibody CLBFcRgranI and CLB-LM6.30 were generously provided by Dr. M. de Haas (CLB, Amsterdam, The Netherlands), and GRM1 was generously provided by Dr. E Garrido (Hospital Virgen de las Nieves, Granada, Spain).

### (Statistical analyses)

Differences in total sFcγRIII, sFcγRIIIa and sFcγRIIIa^{Mφ} levels and laboratory data between two groups were tested by analysis of variance (ANOVA) with Fisher's PLSD post hot test, and correlation were tested by a Bartlett's test.

### Reference Example 1.

### Preparation of FcγRIIIa^{Mφ}

FcγRIIIa^{Mφ} was purified from NP-40 (poly(oxyethylene)(9) octylphenyl ether from Wako Pure Chemical Industries, Ltd.) lysate of 4 day-cultured monocytes.

Briefly, monocytes were isolated from a buffy coat prepared from citrated blood of healthy donoers by Perooll density centrifugation and subsequent counterflow centrifugal elutriation of the mononuclear leukocytes according to a method of Masuda et aL (J. Immunol.,151, 7188-7195, 1993). The purified monocytes were cultured for 4 days in Iscove's modified Dulbecco modified medium (IMDM, GIBCO) containing 10% fetal calf serum (FCS). The cultured monocytes were lysed at 4°C by treatment for 15 minutes with 1% NP-40 in 110 mM NaCl and 50 mM Tris buffer (pH 7.5), in the presence of 50 µg/L of phenylmethanesulfonyltluoride (PMSF), 1 mM Na-p-tosyl-L-lysine chloromethyl ketone and 40 µg/mL of a soybean trypsin inhibitor. The obtained lysate were precleared with affinity chromatography using Lentil Lectin-Sepharose (from Amersham Phaanacia Biotech AB), then FcγRIIIa^{Mφ} were purified by affinity chromatography with Sepharose CL-4B beads bound with anti-FcγRIII monoclonal antibody CLBFcRgranI.

### Reference Example 2.

### Preparation of an anti FcγRIIIa^{Mφ} monoclonal antibody

A mixture of 0.1 mL PBS solution of FcγRIIIa^{Mφ} purified in Reference Example 1 (an amount 0.1 mg/mL) with a Freund's incomplete adjuvant was intraperitoneally immunized to Balb/c mice (6 weeks old, female) once a week for 3 times. After 4 days from the third immunization, spleens thereof were collected. Hybridomas were prepared according to a conventionally known method with spleen cells and NS-I mouse myeloma cells. Specificity against FcγRIIIa^{Mφ} of the monoclonal antibody was tested by a general ELISA method. Simultaneously, reactivity for neutrophils, NK cells, monocytes, cultured monocytes and intraperitoneal monocytes/macrophages were analyzed by indirect immunofluorescence method. As a result, hybridoma MKGR14 which produce an anti-FcγRIIIa^{Mφ} monoclonal antibody was obtained, and anti-FcγRIIIa^{Mφ} monoclonal antibody MKGR14 produced by the hybridoma was selected as an FcγRIIIa^{Mφ} specific antibody.

The above hybridoma MKGR14 was deposited in International Patent Organism Depositary, National Institute of Advanced Industrial Science and Technology on January 29, 2002 as accession number of PERM BP-7866.

### Reference Example 3.

### Evaluation of specificity of an anti FcγRIIIa^{Mφ} monoclonal antibody

### (1) Flow cytometric analysis

FITC labeled MKGR14 and FITC-labeled CLBFcRgranI were incubated on ice with 4-day cultured monocytes, peripheral leukocytes or peritoneal cells, and then analyzed by flow cytometry.

### (2) Immunoprecipitation analysis

Each of 4-day cultured monocytes, neutrophils and NK cells were lysed by treating with 1% NP-40 (in 110 mM NaCl and 50 mM Tris buffer with pH of 7.5) in the presence of 50 µg/L of phenylmethanesulfbnylfluonde (PMSF), 1 mM Na-p-tosyl-L-Iysine chloromethyl ketone and 40 µg/mL of a soybean trypsin inhibitor. The obtained lysate were incubated with Sepharose CL-4B beads coupled with the anti-FcγRIIIa^{Mφ} monoclonal antibody MKGR14, or Protein G Sepharose 4 Fast Flow beads (from Amersham Pharmacia Biotech AB) coupled with anti-FcγRIII monoclonal antibody CLBFcRgranI, followed by reacting at 4°C for 1 hour. Subsequently, these beads were washed with 1 % NP-40 (in 110 mM NaCl, 50 mM Tris buffer with pH of 7.5) in the presence of 50 µg/L of phenylmethanesulfonylfluoride (PMSF), 1 mM Na-p-tosyl-L-lysine chloromethyl ketone and 40 µg/mL of a soybean trypsin inhibitor, and then, suspended in a SDS sample buffer without reducing agent and were heated at 65°C for 5 minutes to be subjected to SDS-PAGE analysis. After electrophoresis, proteins in the gel were transferred to nitrocellulose membrane, and then reacted with anti-FcγRIII monoclonal antibody CLB-LM6.30 and peroxidase labeled anti-mouse IgG (from Jackson Immuno Research Laboratories, Inc.) sequentially. Peroxidase activity on this membrane was detected with an ELC detection system (from Boehringer Mannheim GmbH, Germany).

### (3) Result

From the results of the above flow cytometric analysis, anti-FcγRIIIa^{Mφ} monoclonal antibody MKGR14 bound to cultured monocytes and peritoneal macrophage, but not react with neutrophils and NK cells. On the other hand, anti-FcγRIII monoclonal antibody CLBFcRgranI reacted with all cell species examined.

Further, from the results of above Immunoprecipitation analysis, anti-FcγRIIIa^{Mφ} monoclonal antibody MKGR14 reacted with FcγRIIIa^{Mφ} expressed on cultured monocytes, but not reacted with FcγRIII expressed on NK cells and neutrophils. On the other hand, anti-FcγRIII monoclonal antibody CLBFcRgranI was reacted with all FcγRIIIs on monocytes, NK cells and neutrophils.

All these results showed that anti-FcγRIIIa^{Mφ} monoclonal antibody MKGR14 specifically recognizes FcγRIIIa^{Mφ}.

### Example 1.

### Comparison of amounts of sFcγRIIIs in plasmas from healthy subjects and patients with CAD

### (1) Measurement of total sFcγRIII

An amount of total sFcγRIII in plasma from healthy subjects and patients with CAD were measured with ELISA.

Briefly, an ELISA plate with 96 wells was coated with anti-FcγRIII monoclonal antibody CLBFcRgranI. After unbound sites had been blocked with 2 % milk in phosphate buffered saline (PBS), 100 µL of EDTA-plasma diluted with High Performance Elisa buffer (HPE buffer, from CLB Inc., Amsterdam, The Netherlands) was incubated in the wells for 1 hour at room temperature. After washing with PBS containing 0.05 % Tween 20, 100 µL of biotin-labeled rabbit anti-FcγRIII monoclonal antibody were added in the wells, incubated. The rabbit anti-FcγRIII antibody used for biotin-labeling was obtained by immunizing a rabbit with FcγRIIIb purified from neutrophils according to the conventional method. After incubating with 100 µL of horse-radish peroxidase-labeled streptavidin, the amount of total sFcγRIII was detected with tetramethylbenzidine and H₂O₂.

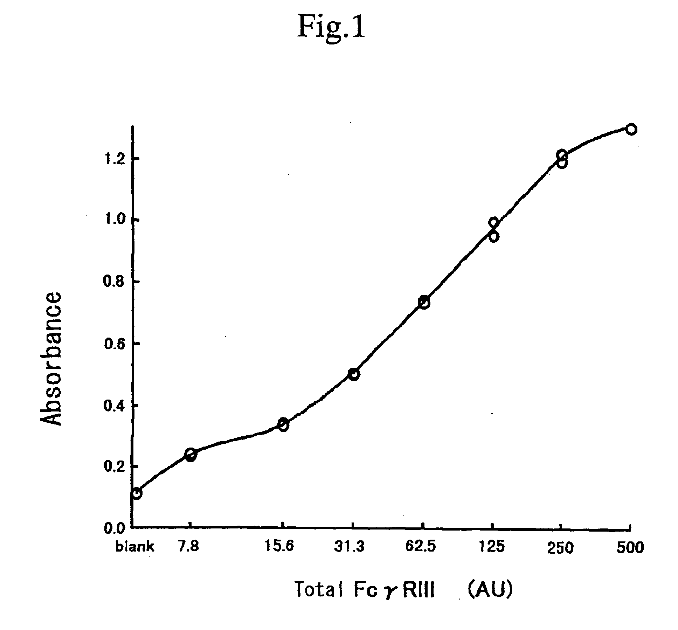

Fig. 1 shows a calibration curve prepared by plotting absorbance on the ordinate and the amount of total sFcγRIII (AU) on the abscissa.

### (2) Measurements of sFcγRIIIa and sFcγRIIIa^{Mφ}

Amounts of sFcγRIIIa and sFcγRIIIa^{Mφ} in plasmas from healthy subjects and patients with CAD were measured with Immuno-PCR assay.

Biotinylated DNA (227 bp) has been produced by PCR amplification with Perkin-Elmer Cetus 9600 instrument (from Perkin-Elmer Japan CO., Ltd.) with a biotin labeled M13-20 primer (biotin-5'-GTAAAACGACGGCCAGT-3'; SEQ ID NO: 1) and a non-labeled M13 reverse primer (5'-GGAAACAGCTATGACCATG-3'; SEQ ID NO: 2) and a plasmid Blue script (2.96 kB, from Toyobo Inc.) as a template. After amplification of 30 cycles, PCR products were purified with CHROMASPIN TE-200 column (from Clontech Laboratories Inc., Palo Alto, CA).

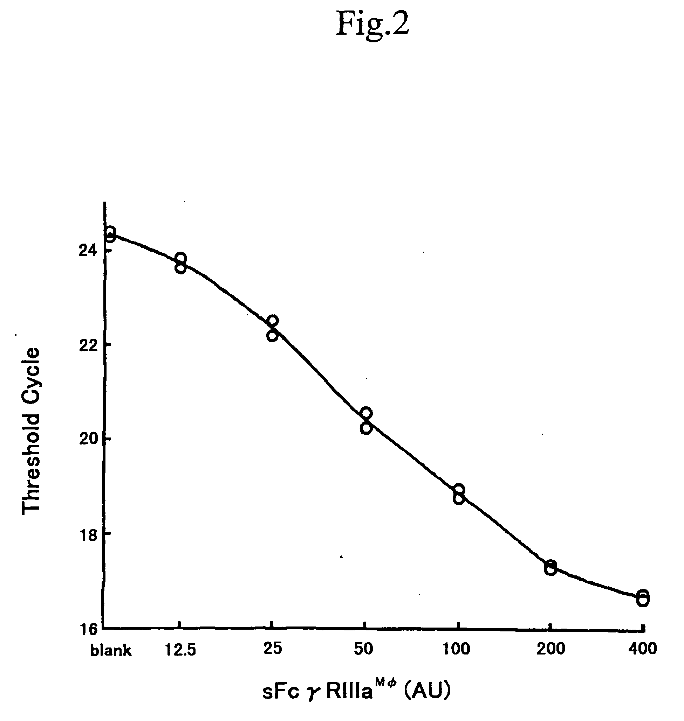

Thin-walled 96-well polypropylene plates suitable for thermocycling (from Bio Medical Equipment) was coated with anti-FcγRIII monoclonal antibody GRM1 for sFcγRIIIa or with anti-sFcγRIIIa^{Mφ} monoclonal antibody MKGR14 for sFcγRIIIa^{Mφ}. After unbound sites were blocked with 1 g/L salmon sperm DNA, 1 % FCS, 5% milk and 1 % gelatin in PBS, 20 µL of EDTA-plasma diluted with HPE buffer was incubated in the wells for overnight at 4°C. After washing 5 times with PBS containing 0.05% Tween 20, the plates were incubated with biotin-labeled anti-FcγRIII monoclonal antibody CLBFcRgranI for sFcγRIIIa or GRM1 for sFcγRIIIa^{Mφ}. After washing 5 times, the plates were incubated with 1 mg/L of avidin (ImmunoPure® NeutrAvidin®, from Pierce Inc., HPE buffer containing 1 g/L salmon sperm DNA) at room temperature for 1 hour. After washing 5 times, the plates were incubated with biotinylated DNA 5 nM (in HPE buffer containing 1 g/l salmon sperm DNA) prepared in the above at room temperature for 1 hour. The amount of sFcγRIII was detected by real time PCR with an ABI PRISM® 7700 Sequence Detection System (from Applied Biosystems Japan KK). Fig. 2 shows a calibration curve which prepared by plotting a threshold cycle (number of cycles to a constant amplified product, Ct value) on the ordinate and an amount of sFcγRIII^{Mφ} (AU) on the abscissa.

### (3) Results

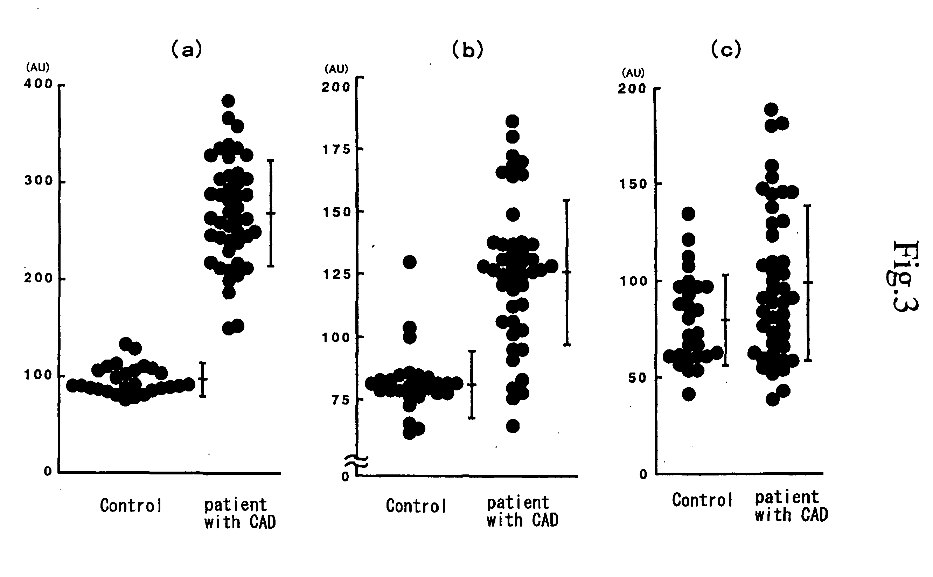

An amount of sFcγRIIIa^{Mφ} in pooled plasma from healthy subjects was set as reference value, i.e. 100 arbitrary units (AU), and the value was compared with the amount of sFcγRIIIa^{Mφ} in plasma from healthy subject or patient with CAD. Results were shown in Fig. 3(a). Similarly, the amounts of sFcγRIIIa and total sFcγRIII in pooled plasma from healthy subject were set as 100 AU, and the value were compared with the amount of sFcγRIIIa or total sFcγRIII in plasmas from healthy subject, or compared with those from patients with CAD. Results were shown in Fig. 3(b) and Fig. 3(c), respectively. Significant level of the value of patient with CAD to the value of healthy control is p<0.05 in Fig. 3(c), and p<0.01 in Fig. 3(a) and Fig. 3(b), respectively.

As shown in Fig. 3, the amount of total sFcγRIII (sFcγRIIIa + sFcγRIIIb) and the amount of sFcγRIIIa (sFcγRIIIa^{Mφ} + sFcγRIIIa^{NK}) were higher in patients with CAD than healthy control (Fig. 3(b) and Fig. 3(C)). The level of sFcγRIIIa^{Mφ} in patients with CAD was about 3 times higher than that in healthy control, and the range of the amounts of sFcγRIIIa^{Mφ} between both groups was not overlapped (Fig. 3(a)).

From these results, an amount of sFcγRIIIa^{Mφ}, in particular, can be used as a marker in diagnosis of atherosclerosis.

### Example 2.

### Measurement-1 of an amount of sFcγRIIIa^{Mφ} in plasmas from patients with CAD

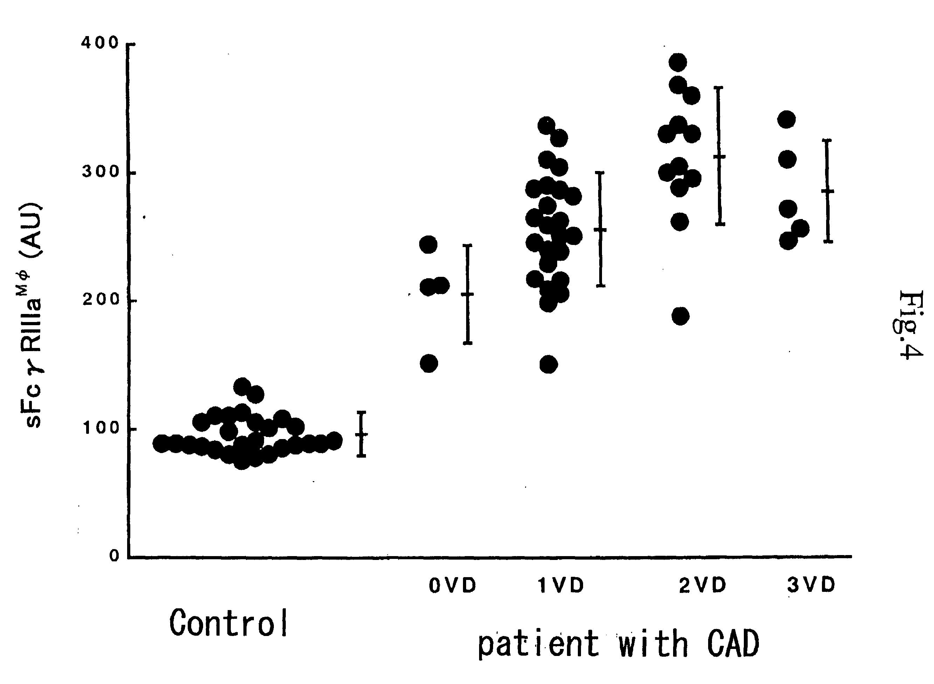

Measurement results of the amount of sFcγRIIIa^{Mφ} in plasma from patients with CAD obtained in Example 1 (Fig. 3 (a)) were classified into 4 groups, i.e. no stenosis (no vessel disease (0 VD)), one-vessel disease (1 VD), two-vessel disease (2 VD) and three-vessel disease (3 VD) according to the number of significant artery stenosis. Results were shown in Fig. 4. In Fig. 4, a significant level of the values inpatients from 0 VD to 3 VD against values of healthy control was p<0.01, significant level of the value of 1 VD to the value of 0 VD was p<0.05, significant level of the values of 2 VD and 3V D to the value of 0 VD were p<0.01, and significant level of the value of 2 VD to value of 1 VD was p<0.01.

As shown in Fig. 4, the sFcγRIIIa^{Mφ} level of patients with CAD showed higher in patients with more number of significant artery stenosis.

From above results, the amount of sFcγRIIIa^{Mφ} reflects severity of CAD.

### Example 3.

### Effect of aging

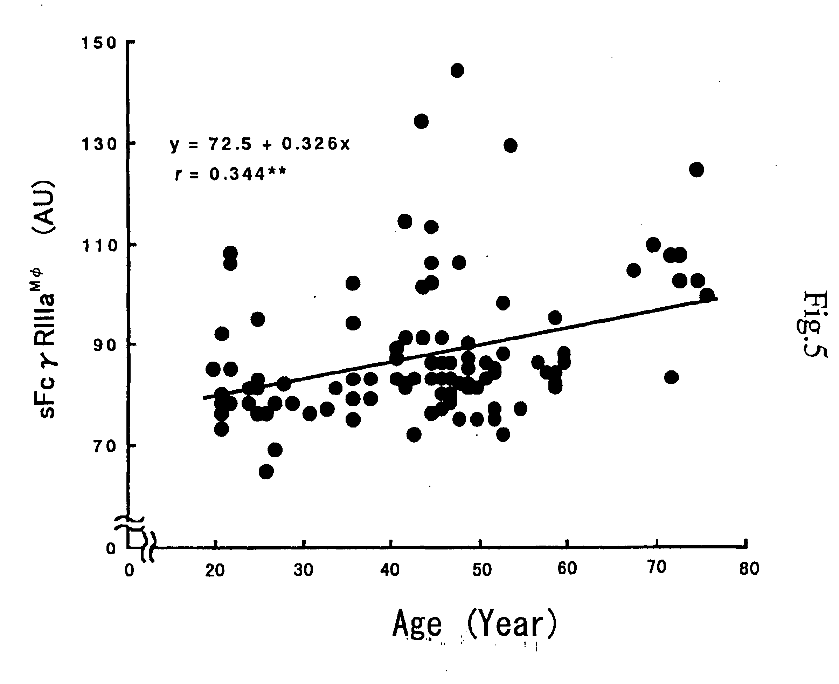

Effect of aging to an amount of sFcγRIIIa^{Mφ} in plasma from healthy subject (AU) which measured by similar manner as in Example 1 was shown in Fig. 5.

As is clear from Fig. 5, an amount of sFcγRIIIa^{Mφ} in plasma increased with age, even though a subject who look like healthy (n = 107, y = 725 + 0.326x, r = 0.344). High amounts of sFcγRIIIa^{Mφ} in plasma were also observed even in young generation. As mentioned above, an amount of sFcγRIIIa^{Mφ} in plasma increased with age, but the degree of increase was individually diffrences. For Example, subjects having higher amount of sFcγRIIIa^{Mφ} in plasma or those having increased tendency even in the young generation are relatively progressing atherosclerosis in the younger stage, there are high risk of development of atherosclerotic complication. Consequently, the risk of future development of atherosclerotic complication can be determined by measuring an amount of sFcγRIIIa^{Mφ} in plasma.

### Example 4.

### Measurement-2 of an amount of sFcγRIIIa^{Mφ} in plasma from a patient with CAD

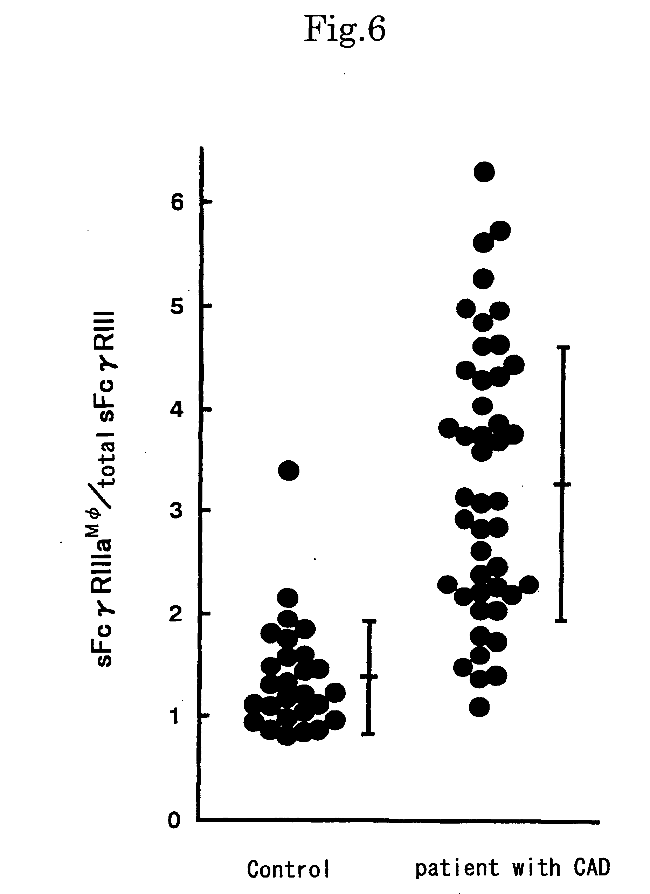

Ratios of the amount of sFcγRIIIa^{Mφ} to the total amount of sFcγRIII (a ratio of sFcγRIIIa^{Mφ} / total sFcγRIII) were calculated by using the values of sFcγRIIIa^{Mφ} and total sFcγRIII in plasmas from healthy control and patients with CAD which were obtained in Example 1. The results were shown in Fig. 6. As shown in Fig. 6, the significant level of the value in patients with CAD to the value of healthy control is p<0.01.

As shown in Fig. 6, ratios of sFcγRIIIa^{Mφ}/total sFcγRIII of patients with CAD were significantly higher than that of healthy control. Consequently, it can be understood that the ratios of sFcγRIIIa^{Mφ}/total sFcγRIII can be a criterion for determining a risk of atherosclerosis, and a risk of future development of atherosclerotic complications.

### Example 5.

### Measurement-3 of an amount of sFcγRIIIa^{Mφ} in plasmas from patients with CAD

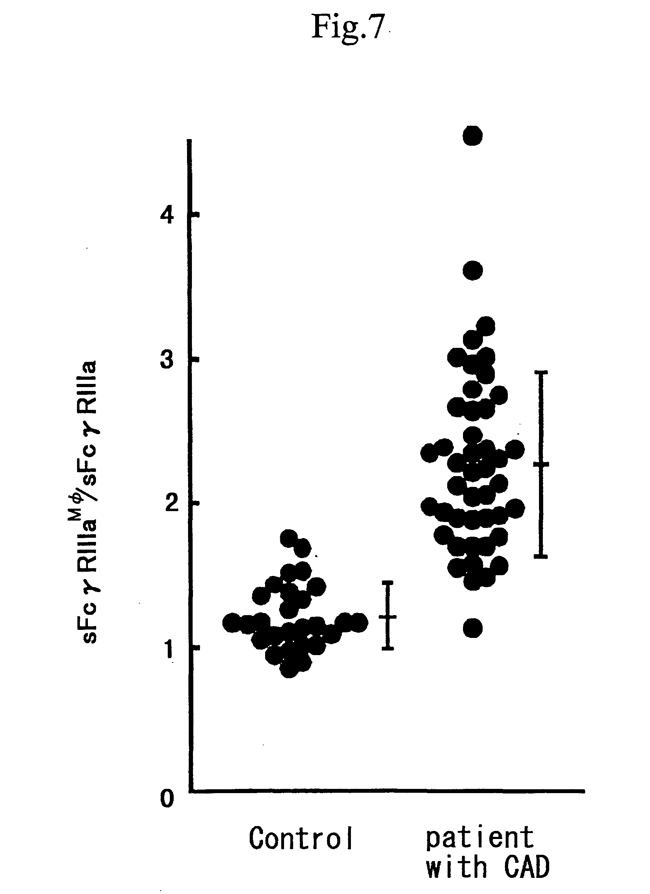

Ratios of the amount of sFcγRIIIa^{Mφ} to the amount of sFcγRIIIa (ratio of sFcγRIIIa^{Mφ} / sFcγRIIIa) were calculated by using the value of sFcγRIIIa^{Mφ} and sFcγRIIIa in plasmas from healthy control and patients with CAD, which was obtained in Example 1. The results were shown in Fig. 7. As shown in Fig. 7, significant level of the value in patients with CAD to the value of healthy control was p<0.01.

As shown in Fig. 7, ratios of sFcγRIIIa^{Mφ}/sFcγRIIIa in patients with CAD were significantly higher than that of healthy control. Consequently, it can be understood that the ratio of sFcγRIIIa^{Mφ} / sFcγRIIIa can be a criterion for determining a risk of atherosclerosis, and future development of atherosclerotic complications.

### Example 6.

### Measurement-4 of an amount of sFcγRIIIa^{Mφ} in plasmas of patients with CAD

Effect of diabetes mellitus accompanied by progress of atherosclerosis on an amount of sFcγRIIIa^{Mφ} in plasmas in patients with CAD was studied.

Patients of 137 of Example 1, who were diagnosed as CAD without any evidence of renal, hepatic, infectious and inflammatory disease, were classified into a group having normal blood sugar (n = 77, 61.9 ± 9.8 years old) and a group complicated with diabetes mellitus (n = 60, 63.1 ± 10.1 years old). NA phenotype of patients was not considered. Plasmas from healthy control were collected without considering NA phenotype (n = 78, 59.8 ± 6.7 years old).

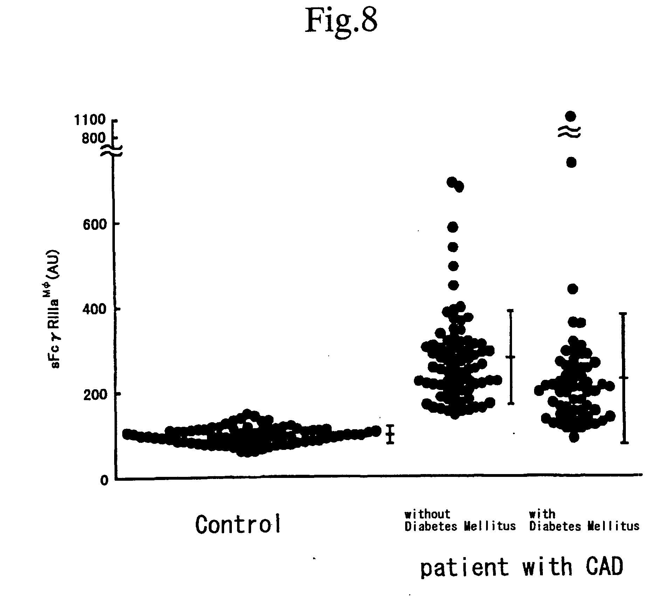

An amount of sFcγRIIIa^{Mφ} in plasma was measured by a similar method as in Example 1 (2). Results are shown in Fig. 8. As shown in Fig. 8, significant level of the value in the group of CAD patients without diabetes mellitus and the value of the group of CAD patients with diabetes mellitus to the value of healthy controls were both p<0.01, and significant level of the value of the group with diabetes mellitus to the value in the group of CAD patients without diabetes mellitus was p<0.01.

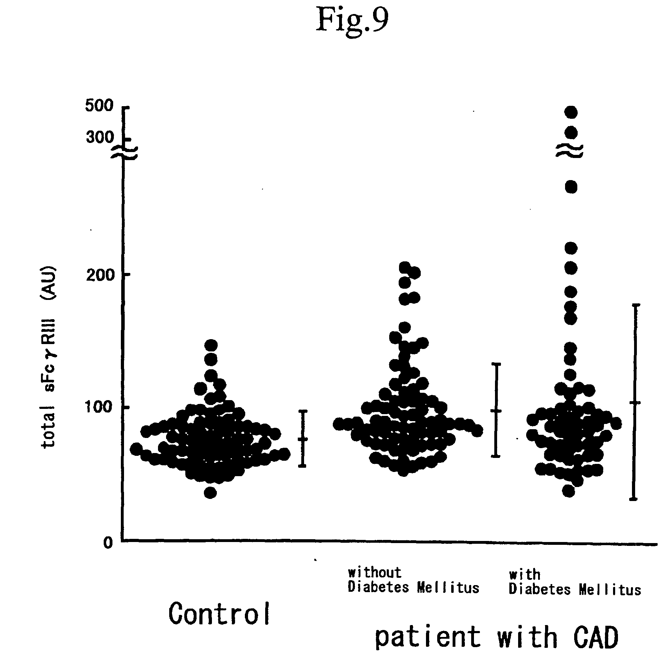

Total amount of sFcγRIII in each plasma was measured according to a similar method as in Example 1 (1). Results were shown in Fig. 9. As shown in Fig. 9, significant level of the value of the group of CAD patient without diabetes mellitus and the value of the group of CAD patients with diabetes mellitus to the value of healthy controls were both p<0.01, and there was no significant difference in the value in the group of CAD patients with diabetes mellitus to the value of the group of CAD patients without diabetes mellitus.

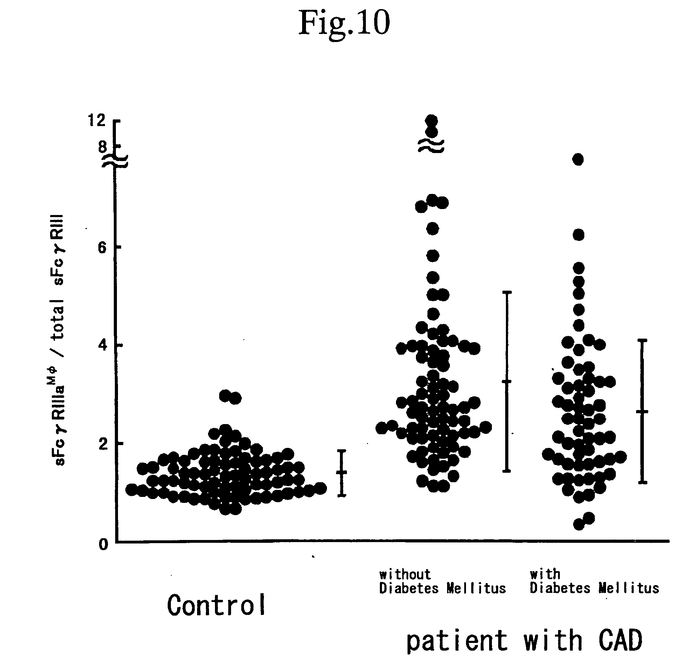

The ratio of the amount of sFcγRIIIa^{Mφ} to the amount of total sFcγRIII (ratio of sFcγRIIIa^{Mφ} / total sFcγRIII) was determined in each of healthy control, the group of CAD patients without diabetes mellitus and the group of CAD patients with diabetes mellitus. Results were shown in Fig. 10. In Fig. 10, significant level of the value of the group of CAD patients without diabetes mellitus and the value of the group of CAD patients with diabetes mellitus to the value of healthy controls were p<0.01, and the significant level of the value of the group of CAD patients with diabetes mellitus to the value of the group of CAD patients without diabetes mellitus was p<0.05.

As shown in Fig. 8, an amount of sFcγRIIIa^{Mφ} in patients with CAD, was higher than that of healthy control, and there was significant differences in the amount of sFcγRIIIa^{Mφ} of healthy control between in both of the group of CAD patients with/without diabetes mellitus. However, when the amounts of sFcγRIIIa^{Mφ} of the group in patients with CAD without diabetes mellitus is compared with the amount of the group in patients with CAD with diabetes mellitus (the group complicating diabetes mellitus), only slight increase was observed in the group of CAD patients with diabetes mellitus.

As shown in Fig. 9, although lower increasing tendency of an amount of sFcγRIII is recognized in a group of CAD patients with diabetes mellitus to a group of CAD patients without diabetes mellitus, it can be understood that difference between the groups from healthy controls were not so large.

On the other hand, as is clear from Fig. 10, when the groups were compared by ratio of sFcγRIIIa^{Mφ} / total sFcγRIII, both groups of CAD patients with/without diabetes mellitus showed significantly higher values than that of healthy control, and difference caused by complication of diabetes mellitus in patients with CAD became smaller. Namely, difference between the value of the group of the CAD patients without diabetes mellitus and the value of the group with mellitus became smaller.

These results show that a ratio of sFcγRIIIa^{Mφ} / total sFcγRIII is useful for determining a risk of atherosclerosis or future development of atherosclerotic complication.

### INDUSTRIAL APPLICABILITY

The determination method of the present invention significantly mitigates patient burden, compared with a conventional cardiac catheterization method, because it is the determination method by measuring sFcγRIIIa^{Mφ} which is a novel predictive marker of atherosclerosis in a blood sample. Furthermore, the present invention can also determine a risk of a case of superficially healthy but with progressing atherosclerosis internally, that is a risk of coming down with atherosclerotic complications in the future.

In Examples, only the cases of cardiac catheterization (coronary angiography) which can quantify degree of atherosclerosis were described. Other than this, some quantification methods of atherosclerosis are being considered, but there is no defined method at present. That is, Examples are only the cases of coronary atherosclerosis, however, the present method can also be applied for determination of systemic atherosclerosis (for example, cerebral atherosclerosis, nephrosclerosis and atherosclerotic obliteration).

## Claims

1. A method for determining a risk of atherosclerosis or future development of atherosclerotic complications, which comprises;
measuring an amount of soluble Fcγ receptor IIIa in a blood sample from patients by using an antibody specifically recognizing Fcγ receptor IIIa, and
determining the risk on the basis of the result of the measurement.

2. The method according to claim 1, wherein the determination is performed by comparing the result with pre-determined amount, and determining the risk on the basis of the result

3. The method according to claim 2, wherein the pre-determined amount is determined based on the amount of soluble Fcγ receptor IIIa in a blood sample from a healthy subject.

4. The method according to claims 1 to 3, wherein the Fcγ receptor IIIa is a soluble Fcγ receptor IIIa^{Mφ} derived from macrophage.

5. The method according to claims 1 to 4, wherein the antibody specifically recognizing Fcγ receptor IIIa is an antibody specifically recognizing Fcγ receptor IIIa^{Mφ} expressed on macrophage.

6. A method for determining a risk of atherosclerosis or future development of atherosclerotic complications, the method comprising the steps of;
(1) measuring an amount of total soluble Fcγ receptor III in a blood sample from patients by using an antibody specifically recognizing Fcγ receptor III,
(2) measuring an amount of soluble Fcγ receptor IIIa^{Mφ} derived from macrophage in a blood sample from patients by using an antibody specifically recognizing Fcγ receptor IIIa^{Mφ} expressed on macrophage,
(3) calculating a ratio of the amount of soluble Fcγ receptor IIIa^{Mφ} derived from macrophage to the amount of total soluble Fcγ receptor III, and
determining the risk on the basis of the result of calculation.

7. A method for determining a risk of atherosclerosis or future development of atherosclerotic complications, the method comprising the steps of;
(1) measuring an amount of soluble Fcγ receptor IIIa in a blood sample from patients by using an antibody specifically recognizing Fcγ receptor IIIa,
(2) measuring an amount of soluble Fcγ receptor IIIa^{Mφ} derived from macrophage in a blood sample from patients is measured by using an antibody specifically recognizing Fcγ receptor IIIa^{Mφ} expressed on macrophage, and
(3) calculating a ratio of the amount of soluble Fcγ receptor IIIa^{Mφ} derived from macrophage to the amount of soluble Fcγ-receptor IIIa, and
determining the risk on the basis of the result of the calculation.

8. A kit for measuring an amount of soluble Fcγ receptor IIIa^{Mφ} derived from macrophage, which comprises a solid phase coated with an antibody specifically recognizing Fcγ receptor IIIa^{Mφ} expressed on macrophage, and a labeled antibody specifically recognizing Fcγ receptor III or Fcγ receptor IIIa.

9. A kit for measuring an amount of soluble Fcγ receptor IIIa^{Mφ} derived from macrophage and an amount of total soluble Fcγ receptor III, which comprises a solid phase coated with an antibody specifically recognizing Fcγ receptor IIIa^{Mφ} expressed on macrophage, a labeled antibody specifically recognizing Fcγ receptor III or Fcγ receptor IIIa, a solid phase coated with an antibody specifically recognizing Fcγ receptor III, and a labeled antibody specifically recognizing Fcy receptor III.

10. A kit for measuring an amount of soluble Fcγ receptor IIIa^{Mφ} derived from macrophage and an amount of soluble Fcγ receptor IIIa, which comprises a solid phase coated with an antibody specifically recognizing Fcγ receptor IIIa^{Mφ} expressed on macrophage, a labeled antibody specifically recognizing Fcγ receptor III or Fcγ receptor IIIa, and a solid phase coated with an antibody specifically recognizing soluble Fcγ receptor IIIa.

11. A kit for determining a risk of atherosclerosis or future development of atherosclerotic complications, which comprises a solid phase coated with an antibody specifically recognizing Fcγ receptor IIIa^{Mφ} expressed on macrophage, and a labeled antibody specifically recognizing Fcγ-receptor III or Fcγ receptor IIIa.

12. A kit for determining a risk of atherosclerosis or future development of atherosclerotic complications, which comprises a solid phase coated with an antibody specifically recognizing Fcγ receptor IIIa^{Mφ} expressed on macrophage, a labeled antibody specifically recognizing Fcγ receptor III or Fcγ receptor IIIa, and a solid phase coated with an antibody specifically recognizing Fcγ receptor III.

13. Akit for determining a risk of atherosclerosis or future development of atherosclerotic complications, which comprises a solid phase coated with an antibody specifically recognizing Fcγ receptor IIIa^{Mφ} expressed on macrophage, a labeled antibody specifically recognizing Fcγ receptor III or Fcγ receptor IIIa, and a solid phase coated with an antibody specifically recognizing Fcγ receptor IIIa.


SEQUENCE LISTING

<110> Wako Pure Chemical Industries, Ltd.

<120> Method for determining atherosclerosis

<130> atherosclerosis

<140>
<141>

<160> 2

<170> PatentIn Ver. 2.1

<210> 1
<211> 17
<212> DNA
<213> Artificial Sequence

<220>
<221> misc_binding
<222> (1)

<400> 1
gtaaaacgac ggccagt 17

<210> 2
<211> 19
<212> DNA
<213> Artificial Sequence

<400> 2
ggaaacagct atgaccatg 19